# EUROPÄISCHE PATENTSCHRIFT

(11) **EP 2 210 106 B1**
(45) Veröffentlichungstag und Bekanntmachung des Hinweises auf die Patenterteilung: **19.09.2012**
(21) Anmeldenummer: 08849463.8
(22) Anmeldetag: 07.11.2008
(51) Int. Cl.: A61K 35/28, G01N 33/574, B82Y 30/00, G01N 33/569

(54) **Verwendung von nanostrukturierten Oberflächen und Verfahren zum Anreichern oder Isolieren von zellulären Subpopulationen**
Use of nanopatterned surfaces and method for enriching or isolating cellular subpopulations
Utilisation de surfaces nanostructurées, et procédé d'enrichissement ou d'isolation de sous-populations cellulaires.

(30) Priorität: 14.11.2007 DE 102007054691
(43) Veröffentlichungstag der Anmeldung: 28.07.2010
(73) Patentinhaber: Leibniz-Institut für Neue Materialien gemeinnützige GmbH, 66123 Saarbrücken (DE)
(72) Erfinder: VEITH, Michael, 66386 St. Ingbert (DE); NARZ, Frank, 58456 Witten (DE)
(74) Vertreter: Vièl, Christof
(86) Internationale Anmeldenummer: PCT/DE2008/001825
(87) Internationale Veröffentlichungsnummer: WO 2009/062470

(56) Entgegenhaltungen:
- KEON WOO KWON ET AL: "Label-Free, Microfluidic Separation of Human Breast Carcinoma and Epithelial Cells by Adhesion Difference" SOLID-STATE SENSORS, ACTUATORS AND MICROSYSTEMS CONFERENCE, 2007. TRANSDUCERS 2007. INTERNATIONAL, IEEE, PISCATAWAY, NJ, USA, 1. Juni 2007 (2007-06-01), Seiten 699-702, XP031133286 ISBN: 978-1-4244-0841-2
- DEWEZ J-L ET AL: "Adhesion of mammalian cells to polymer surfaces: from physical chemistry of surfaces to selective adhesion on defined patterns" BIOMATERIALS, ELSEVIER SCIENCE PUBLISHERS BV., BARKING, GB, Bd. 19, Nr. 16, 1. August 1998 (1998-08-01), Seiten 1441-1445, XP004161407 ISSN: 0142-9612
- ARONOV ET AL: "Hydroxyapatite nanoceramics: Basic physical properties and biointerface modification" JOURNAL OF THE EUROPEAN CERAMIC SOCIETY, ELSEVIER SCIENCE PUBLISHERS, BARKING, ESSEX, GB, Bd. 27, Nr. 13-15, 1. Januar 2007 (2007-01-01), Seiten 4181-4186, XP022143719 ISSN: 0955-2219

## Beschreibung

Die Erfindung betrifft die Verwendung von nanostrukturierten Oberflächen. Sie betrifft ebenfalls ein Verfahren zum Anreichern oder Isolieren von zellulären Subpopulationen.

Das Anreichern oder Isolieren von zellulären Subpopulationen aus einer komplexen Mischung (z.B. T-Zellen aus Blut) ist ein bedeutender Schritt bei der voranalytischen Probenzubereitung. Es erfolgt derzeit unter Verwendung von Antikörpern, die gegen ein bestimmtes Oberflächenprotein gerichtet sind und magnetischen Partikeln, um die gebundenen Zellen aus der Probe zu entefernen, was jedoch mit folgenden Nachteilen verbunden ist:
- Die interessierenden Zellen müssen ein spezifisches Oberflächenprotein ausdrücken.
- Ein Antikörper gegen dieses Protein muß zur Verfügung stehen.
- Die Aufbewahrung der Antikörper ist problematisch.
- Antikörper sind teuer.
- Beim Binden des Antikörpers an das Oberflächenprotein kann die Zellphysiologie verändert werden.

Statt der magnetpartikelbasierten Isolierung können auch fluoreszierende Antikörper in Verbindung mit einem FACS (fluorescence associated cell sorter, fluoreszenzbasierter Zellsortierer) verwendet werden. Auch hier stellt sich das Problem der Kosten und zudem das der komplexen apparativen Mittel.

Weiter können physikalische Parameter, wie beispielsweise die Zelldichte (HEXAL Oncoquick, *http:*//*wwww.hexal-gentech.com*/*products*/*man e.pdf)* oder die Zellgröße *(*Am. J. Pathol, 2000, (1), 156, 5 7-63*, Isolation by Size of Epithelial Tumor Cells, Giovanna Vona et. al*.) verwendet werden. Da jedoch nicht jeder Zelltyp charakteristische physikalische Parameter aufweist, ist dies nur möglich, wenn die interessierenden Zellen charakteristische physikalische Parameter aufweisen.

Aufgabe der Erfindung ist es, ein einfaches, vielseitig verwendbares und spezifisches Verfahren zum Anreichern oder Isolieren von zellulären Subpopulationen aus einer komplexen Mischung zu schaffen.

Die Verwendung von nanostrukturierten Oberflächen zur Isolierung und Anreicherung von zellulären Subpopulationen aus einer komplexen Mischung ist beispielsweise aus der Druckschrift *"*Label-Free, Microfluidic Separation of Human Breast Carcinoma and Epithelial Cells by Adhesion Difference" (Kwon et al., Transducers and Eurosensors 2007, conference proceedings) bekannt. Die Anreicherung der Zellen wird mit Hilfe einer nanostrukturierten Oberfläche in einem mikrofluiden System erzielt, wobei die Oberfläche aus 50 nm großen Säulen aus PDMS auf einer Glasoberfläche besteht.

Es hat sich im Rahmen der Erfindung überraschend gezeigt, dass auf Oberflächen bestehend aus eindimensionaler Nanodrähter ist beispielsweise bestimmte zelluläre Subpopulationen selektiv und in bisher nicht bekannter Effizienz deutlich besser als andere Substrate anhaften.

In einer besonders bevorzugten Ausführungsform bestehen solche nanostrukturierten Oberflächen aus eindimensionalen Nanodrähten, wie sie in der DE 10 2006 013 484 A1 beschrieben sind. Hierbei bestehen die Nanodrähte aus einem metallischen Kern und sind ummantelt mit einer keramischen Hülle und eignen sich in besonderer Weise zum Anreichern oder Isolieren von zellulären Subpopulationen aus einer komplexen Mischung erreicht.

Es liegt im Rahmen der Erfindung, dass die verzweigten Nanostrukturen eindimensionale Kompositstrukturen mit zwei Dimensionen im Submikrometerbereich sind und diese Kompositstrukturen aus dem Kern eines Materials, insbesondere Metall, und einer Hülle eines anderen Materials, insbesondere aus Keramik, aufgebaut sind und durch thermolytische Zersetzung von Verbindungen der allgemeinen Struktur El(OR)H₂,
- in der El die Elemente Al, Ga, In und Tl bedeutet und
- R für ein Alkyl (C3-C10) oder ein Cycloalkyl (C5-C8) steht,
gemäß der DE 10 2006 013 484 A1 herstellbar sind.

Es liegt im Rahmen der Erfindung, dass die zellulären Subpopulationen bestimmte humane oder veterinäre Zellen sind und dass die komplexe Mischung Plasma ist.

Eine Ausbildung der Erfindung besteht darin, dass das Plasma Humanplasma ist.

Eine bevorzugte Ausbildung der Erfindung besteht darin, dass die selektiv adsorbierten Blutzellen nicht intrinsisch anhaftende Zellen, insbesondere Jurkatzellen sind.

Unter nicht intrinsich anhaftenden Zellen werden solche Zellen verstanden, die normalerweise in einer Suspension leben. Das Kultivieren solcher Zellen auf einem Substrat ist in der Regel sehr problematisch.

Im Rahmen der Erfindung liegt auch ein Verfahren zum Anreichern oder Isolieren von zellulären Subpopulationen, wobei die zellulären Subpopulationen sich an nanostrukturierten Oberflächen anreichern.

In einer besonders bevorzugten Ausführungsform sind die nanostrukturierten Oberflächen aus eindimensionalen Nanodrähten aufgebaut, bestehend aus einem metallischen Kern und ummantelt mit einer keramischen Hülle, wie sie in der DE 10 2006 013 484 A1 beschrieben sind. Auf diesen Strukturen adhärieren Zellen selektiv, reichern sich somit an.

Es liegt im Rahmen der Erfindung, dass die zellulären Subpopulationen bestimmte Blutzellen sind und dass die komplexe Mischung Plasma ist.

Zur Erfindung gehörig ist, dass das Plasma Humanplasma ist.

Schließlich ist es erfindungsgemäß vorgesehen, dass die zellulären Subpopulationen nicht intrinsisch anhaftende Zellen, insbesondere Jurkatzellen sind.

Nachfolgend wird die Erfindung näher anhand von Versuchen in nicht einschränkender Weise erläutert. Die Versuchsergebnisse sind in den Figuren dargestellt.

Es zeigen
- Fig. 1a und Fig. lb: Fluoreszenzmikroskopie-Aufnahmen von verschiedenen Zellkulturen auf verschiedenen Substraten sowie nach Trypsination,
- Fig. 2: eine EDX-Aufnahme der nanokristallinen Oberfläche gemäß der Erfindung.

Beobachtet wurde das Wachstum von eukaryotischen Zellen auf Uhrgläsern, die mit eindimensionalen Kompositstrukturen im Submikrometerbereich beschichtet sind und die einen Kern eines Materials und eine Hülle eines anderen Materials aufweisen

Es wurden runde Uhrgläser (Menzel-Gläser, Deutschland, No. CB00120RA1, 12 mm Durchmesser, 0,13-0,16 mm Stärke, Los Nr. 4710486) verwendet.

Ein Teil dieser Uhrgläser wurden mit einer eindimensionalen Kompositstruktur gemäß der DE 10 2006 013 484 A1 auf Al/Al₂O₃-Basis beschichtet.

Um Verunreinigungen zu entfernen, wurden sowohl die beschichteten als auch die nicht beschichteten Uhrgläser wie folgt behandelt:
- Die Uhrgläser werden (gegebenenfalls mit der beschichteten Seite nach oben) in die Vertiefungen einer Zellkulturplatte mit 24 Plätzen eingebracht
- Es werden 500 µl 70%iger Ethanol zugegeben und 5 min in den Uhrgläsern belassen
- Es wird 3 mal mit je 500 µl sterilem Wasser gespült
- Es wird 3 mal mit je 500 µl sterilem PBS (phosphatgepufferter Salzlösung) gespült

Es werden mit sterilem Poly-Lysin (BD Biosciences, 352085) beschichtete Uhrgläser und nicht hiermit beschichtete Uhrgläser verwendet, wobei erstere nur gewaschen wurden. Zusätzlich wurden Zellen ohne Uhrgläser direkt in der 24-plätzigen Zellkulturplatte kultiviert.

Die Zellen wurden in 1 ml Serum, das Zellkulturmedium enthält, in geeigneter Dichte (typischerweise 4-8 • 10⁴ Zellen pro Vertiefung) aufgegeben und bei 37°C mit 5% CO₂ und 100% Luftfeuchtigkeit kultiviert.

Die Zellen wurden bis zu 72 h kultiviert und täglich mittels eines Lichtmikroskops inspiziert. Anschließend wurden nichtanhaftende Zellen weggewaschen, indem die Vertiefungen und Uhrgläser zweimal mit 1000 µl PBS gespült wurden. Die verbleibenden anhaftenden Zellen wurden durch Zugabe von 500 µl 100%igem Ethanol (5 min, RT) fixiert. Nach dem Entfernen des Ethanols wurden die Zellkerne durch Zugabe von 500 µl Hoechst 33342 (Invitrogen, 0,1% in PBS) gefärbt. Mikroskopische Aufnahmen wurden unter Verwendung eines Fluoreszenzmikroskops gefertigt.

Es wurde die folgende Versuchsreihe durchgeführt:

### Versuch

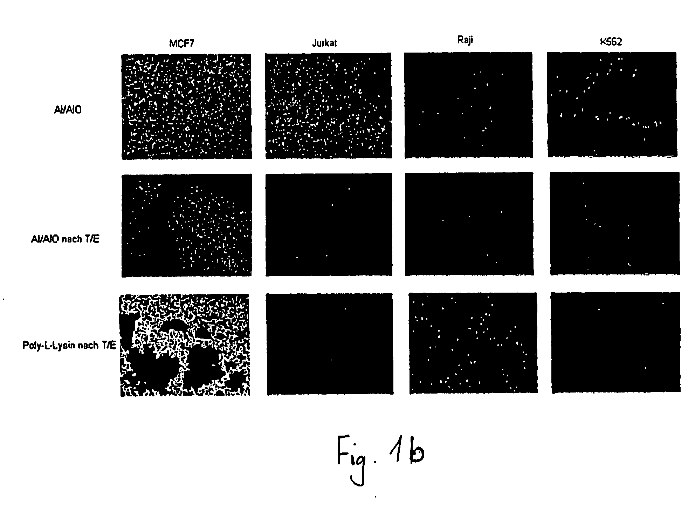

In diesem Versuch wurden (07D.0031 pp38; CK) wurden Jurkat- (humane T-Zell Linie in Suspension), Raji Zellen (humane Macrophagenzellinie in Suspension), K562- (humane B-Zell Linie in Suspension) und MCF7-Zellen (adhärente humane Brustkrebszelllinie) auf Uhrgläsern aus Kunststoff (Bezeichnung in Fig. la: Plastik), auf mit Poly-L-Lysin beschichteten Uhrgläsern (Bezeichnung in Fig. la und 1b: Poly-L-Lysin), auf Uhrgläsern aus Glas sowie auf mit Al/Al₂O₃-beschichteten (Bezeichnung in Fig. 1b: Al/AlO) Uhrgläsern und kultiviert.

Nach 24h wurden die Zellen abgewaschen und die Zellkerne gefärbt, ohne dass die Zellen mit Ethanol fixiert wurden. Mikroskopische Aufnahmen wurden vorgenommen und die Zellen wurden durch Zugabe einer Trypsin/EDTA-Lösung (Bezeichnung in Fig. 1b: nach T/E) (Invitrogen) gelöst. Nach Waschen mit PBS wurden die verbleibenden Zellen erneut photographiert. In diesem Versuch wurde die erhöhte Haftung von Jurkatzellen (als Beispiel nicht intrinsich adhärenter Zellen) auf der Al/AlOₓ-Beschichtung beobachtet. Die anhaftenden Zellen konnten lebend von den Beschichtung durch Trypsination abgelöst werden, was auf eine proteingestützten Haftungsmechanismus hinweist.

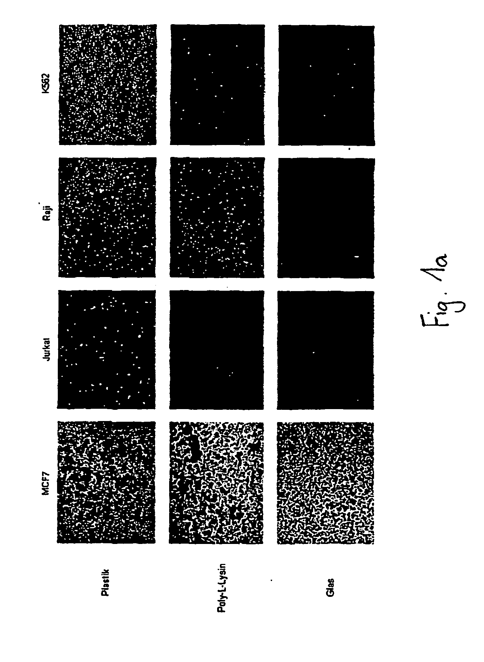

Die Fluoreszenzmikroskopie-Aufnahmen gemäß den Fig. 1a und 1b zeigen, daß die Intensität der Fluoreszenz bei den mit Al/AlOₓ beschichteten Uhrgläsern etwas geringer ist, da die Beschichtung Licht absorbiert. Dennoch ist es offensichtlich, daß auch nicht intrinsisch anhaftende Zellen, wie Jurkatzellen, sich auf den mit Al/AlOₓ beschichteten Uhrgläsern deutlich besser als auf den anderen Substraten anreichern und, wie in weiteren Versuchen festgestellt, auch über längere Zeiträume (mehr als 72 h) voll funktionsfähig überleben. Aufgnmd der Tatsache, daß sich diese Zellen auch lebend ablösen lassen (und somit weiter kultiviert bzw. verarbeitet werden können), kann eine zyklische Anreicherung auch geringster Mengen solcher Zellen auf diesem Substrat erfolgen. Darüber hinaus ist die Zellverteilung gleichmäßig und nicht zu dicht, so daß die Zellmorphologie im Rahmen beispielsweise einer mikroskopischen Untersuchung gut beobachtbar ist, womit eine Stadienbestimmung möglich ist.

Das Anhaften und das Wachstum von intrinsisch adhärenten Zelllinien wie MCF7 wird durch die Al/AlOₓ-Beschichtung nicht gestört.

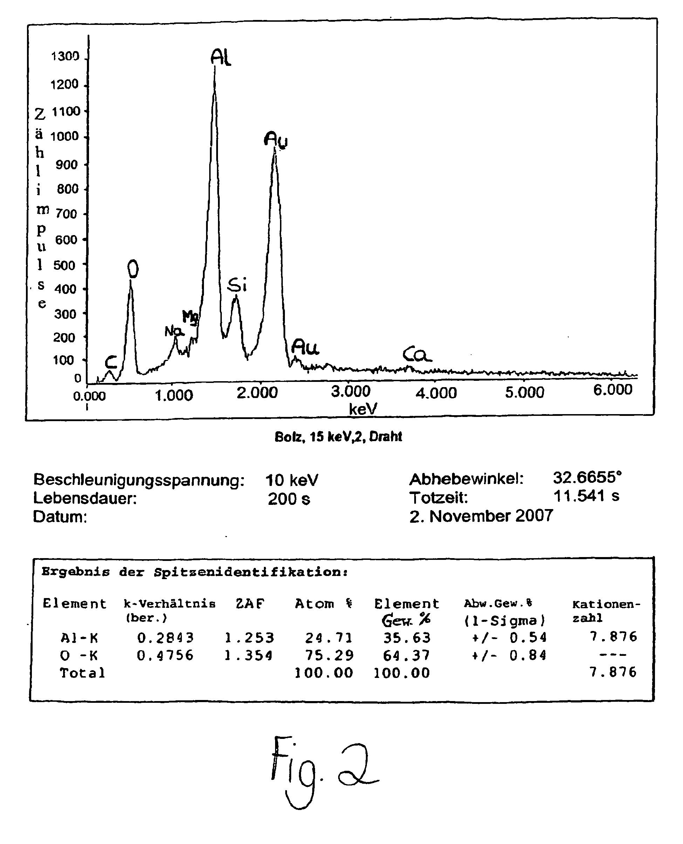

Die EDX-Aufnahme (Fig. 2) der erfindungsgemäßen nanokristallinen Oberfläche zeigt, daß diese keine signifikanten Mengen an Kohlenstoff enthält und somit als anorganisches Substrat anzusehen ist.

## Patentansprüche

1. Verwendung von nanostrukturierten Oberflächen zur Isolierung und/oder Anreicherung von zellulären Subpopulationen aus einer komplexen Mischung, wobei die zellulären Subpopulationen nicht intrinsisch anhaftende Zellen sind, wobei die nanostrukturierten Oberflächen aus eindimensionalen Nanodrähten aufgebaut sind, bestehend aus einem metallischen Kern und ummantelt mit einer keramischen Hülle, wobei der Nanodraht zwei Dimensionen im Submikrometerbereich besitzt und durch thermolytische Zersetzung von Verbindungen der allgemeinen Struktur El(OR)H₂, in der El für die Elemente Al, Ga, In und Tl und R für ein Alkyl (C3-C10) oder ein Cycloalkyl (C5-C8) steht, hergestellt ist.

2. Verwendung gemäß Anspruch 1, **dadurch gekennzeichnet, daß** die zellulären Subpopulationen bestimmte Blutzellen sind und dass die komplexe Mischung Plasma ist.

3. Verwendung gemäß Anspruch 2, **dadurch gekennzeichnet, dass** das Plasma Humanplasma ist.

4. Verfahren zum Anreichern oder Isolieren von zellulären Subpopulationen, **dadurch gekennzeichnet, daß** die zellulären Subpopulationen sich an nanostrukturierten Oberflächen anreichern, wobei die zellulären Subpopulationen nicht intrinsisch anhaftende Zellen sind und wobei die nanostrukturierten Oberflächen aus eindimensionalen Nanodrähten aufgebaut sind, bestehend aus einem metallischen Kern und ummantelt mit einer keramischen Hülle, wobei der Nanodraht zwei Dimensionen im Submikrometerbereich besitzt und durch thermolytische Zersetzung von Verbindungen der allgemeinen Struktur El(OR)H₂, in der El für die Elemente Al, Ga, In und Tl und R für ein Alkyl (C3-C10) oder ein Cycloalkyl (C5-C8) steht, hergestellt werden.

5. Verfahren gemäß Anspruch 4, **dadurch gekennzeichnet, dass** die zellulären Subpopulationen bestimmte Blutzellen sind und dass die komplexe Mischung Plasma ist.

6. Verfahren gemäß Anspruch 5, **dadurch gekennzeichnet, dass** das Plasma Humanplasma ist.

## Claims

1. Use of nanopattemed surfaces for isolating and/or enriching cellular subpopulations from a complex mixture, said cellular subpopulations being intrinsically non-adherent cells and said nanopattemed surfaces being made up of one-dimensional nanowires that consist of a metallic core surrounded by a ceramic shell, have two dimensions in the sub-micrometre range and are produced by thermolytic decomposition of compounds having the general formula El(OR)H₂, where El stands for the elements Al, Ga, In and Tl and R for an alkyl (C3-C10) or a cycloalkyl (C5-C8) group.

2. Use according to claim 1, **characterised in that** the cellular subpopulations are specific blood cells and that the complex mixture is plasma.

3. Use according to claim 2, **characterised in that** the plasma is human plasma.

4. Method for enriching or isolating cellular subpopulations, **characterised in that** the cellular subpopulations are enriched on nanopattemed surfaces, said cellular subpopulations being intrinsically non-adherent cells and said nanopattemed surfaces being made up of one-dimensional nanowires that consist of a metallic core surrounded by a ceramic shell, have two dimensions in the sub-micrometre range and are produced by thermolytic decomposition of compounds having the general formula El(OR)H₂, where El stands for the elements Al, Ga, In and Tl and R for an alkyl (C3-C10) or a cycloalkyl (C5-C8) group.

5. Method according to claim 4, **characterised in that** the cellular subpopulations are specific blood cells and that the complex mixture is plasma.

6. Method according to claim 5, **characterised in that** the plasma is human plasma.

## Revendications

1. Utilisation de surfaces nanostructurées pour l'isolation et/ou l'enrichissement de sous-populations cellulaires à partir d'un mélange complexe, les sous-populations cellulaires n'étant pas des cellules intrinsèquement adhérentes, les surfaces nanostructurées étant construites à partir nanofils unidimensionnels, composés d'un noyau métallique et revêtus d'une enveloppe en céramique, le nanofil possédant deux dimensions dans le domaine submicrométrique et étant fabriqué par décomposition thermolytique de composés de structure générale El(OR)H₂, dans laquelle El représente les éléments Al, Ga, In et Tl et R représente un alkyl (C3-C10) ou un cycloalkyl (C5-C8).

2. Utilisation selon la revendication 1, **caractérisée en ce que** les sous-populations cellulaires sont certaines cellules sanguines et **en ce que** le mélange complexe est du plasma.

3. Utilisation selon la revendication 2, **caractérisé en ce que** le plasma est du plasma humain.

4. Procédé pour enrichir ou isoier des sous-populations cellulaires, **caractérisé en ce que** les sous-populations cellulaires s'accumulent sur les surfaces nanostructurés, les sous-populations n'étant pas des cellules intrinsèquement adhérentes et les surfaces nanostructurées étant construites à partir nanofils unidimensionnels composés d'un noyau métallique et revêtus d'une enveloppe en céramique, le nanofil possédant deux dimensions dans le domaine submicrométrique et étant fabriqué par décomposition thermolytique de composés de structure générale El(OR)H₂, dans laquelle El représente les éléments Al, Ga, In et Tl et R représente un alkyl (C3-C10) ou un cycloalkyl (C5-C8).

5. Procédé selon la revendication 4, **caractérisé en ce que** les sous-populations cellulaires sont certaines cellules sanguines et **en ce que** le mélange complexe est du plasma.

6. Utilisation selon la revendication 5, **caractérisé en ce que** le plasma est du plasma humain.
